(19) Europäisches Patentamt
European Patent Office
Office européen des brevets

(11) Publication number: **0 550 762 B1**

# (12) EUROPEAN PATENT SPECIFICATION

(45) Date of publication of patent specification: **22.11.95**

(51) Int. Cl.[6]: **C07C 253/00**, //B01J23/02, B01J31/02

(21) Application number: **92915837.6**

(22) Date of filing: **21.07.92**

(86) International application number: **PCT/JP92/00928**

(87) International publication number: **WO 93/02046 (04.02.93 93/04)**

## (54) PROCESS FOR PRODUCING NITRILE.

(30) Priority: **25.07.91 JP 186227/91**

(43) Date of publication of application: **14.07.93 Bulletin 93/28**

(45) Publication of the grant of the patent: **22.11.95 Bulletin 95/47**

(84) Designated Contracting States: **AT BE CH DE DK ES FR GB GR IT LI LU MC NL SE**

(56) References cited:
**JP-A- 5 896 053**

**CHEMICAL ABSTRACTS, Vol. 109, No. 9, Abstract No. 109(9) : 72976q, 1988 (Columbus, Ohio, US), K. KIYOTOMI et al., Chem. Lett., (2), 285-6, 1988.**

**CHEMICAL ABSTRACTS, Vol. 105, No. 3, Abstract No. 105(3) : 24029d, 1986 (Columbus, Ohio, US), STENBERG JOHN R., Chem. Ind. (Dekker), 22 (Catal. Org. React.), 373-9, 1985.**

(73) Proprietor: **Kao Corporation**
**14-10, Nihonbashi Kayabacho 1-chome**
**Chuo-Ku**
**Tokyo 103 (JP)**

(72) Inventor: **OKU, Masayuki**
**875-1, Kohdo,**
**Kishigawa-cho**
**Naga-gun,**
**Wakayama 640-04 (JP)**
Inventor: **FUJIKURA, Yoshiaki**
**271-6, Yamamoto-cho**
**Utsunomiya-shi,**
**Tochigi 320 (JP)**

(74) Representative: **Hansen, Bernd, Dr.rer.nat. et al**
**Hoffmann, Eitle & Partner**
**Patentanwälte**
**Postfach 81 04 20**
**D-81904 München (DE)**

EP 0 550 762 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, Vol. 100, No. 1, Abstract No. 100(1) : 6009q, 1984 (Columbus, Ohio, US), A. ORAZIO et al., Synthesis, (9), 741-2, 1983.

CHEMICAL ABSTRACTS, Vol. 82, No. 7, Abstract No. 82(7) : 43035a, 1975 (Columbus, Ohio, US), ROGIC MILORAD M. et al., J. Org. Chem., 39(23), 3424-6, 1974.

## Description

### TECHNICAL FIELD

This invention relates to a process for producing, in high yields and with ease, a nitrile which is useful as a perfume or as a starting material in the synthesis of perfumes, medicines and so on.

### BACKGROUND ART

Several processes are known for obtaining a nitrile from an aldoxime by the use of a dehydrating agent. Typical processes include: (A) a process where an aldoxime is allowed to react with an acid anhydride such as acetic anhydride for dehydration, and (B) a process where an aldoxime is dehydrated with a phosphonitrilic chloride trimer (hexachlorocyclotriphosphazene).

However, process (A) involves disadvantages of high cost of starting material because acetic anhydride is needed in an equimolar amount or more with respect to the amount of aldoxime, and in addition, disposal of waste acetic acid finally produced from the acetic anhydride is required. On the other hand, when process (B) is adopted, phosphonitrilic chloride must be used in an equimolar amount or more with respect to the amount of aldoxime, which means a high cost of starting material, and moreover, waste disposal is even more costly compared to the process utilizing acetic anhydride.

In order to avoid these disadvantages, a process utilizing an acid such as sulfuric acid as a dehydrating agent has been reported (Japanese patent publication kokoku No. 59144/1990). This process is superior to the above-mentioned process in respect of only a catalytic amount of acid and reduced amounts of wastes. But this process is still defective because of the limited application of the process. In detail, yield of 80% or more is assured to obtain a nitrile from an aromatic oxime, a reduced yield of 70% from an aliphatic oxime, and a very low yield of 12% from a terpene oxime, which indicates unsuitableness in the wide-range practice.

Accordingly, an object of this invention is to provide a process for producing a nitrile in high yields and at a remarkably advantageous production cost.

### DISCLOSURE OF THE INVENTION

According to the present invention, there is provided a process for producing a nitrile represented by the general formula (1):

$RC \equiv N$ (1)

wherein R is alkyl, alkenyl, aralkyl or aryl, each may have a substituent, characterizing by heating an aldoxime represented by the general formula (2):

$RCH = NOH$ (2)

wherein R is as defined above, in the presence of one or more catalysts selected from hydroxides of alkali metals, alcoholates of alkali metals, hydroxides of alkaline earth metals and alcoholates of alkaline earth metals, and distilling off water formed in the course of the reaction from the reaction system.

### BEST MODE FOR CARRYING OUT THE INVENTION

This invention will now be described in detail.

This invention is shown by the following reaction scheme:

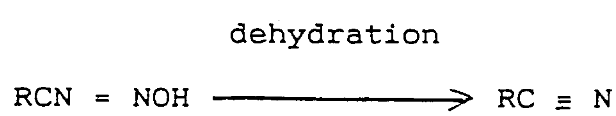


(2) (1)

wherein R has the same meaning as defined above.

In other words, according to the present invention, a nitrile of the general formula (1) is prepared by heating an aldoxime represented by the general formula (2) in the presence of at least one catalyst selected from hydroxides of alkali metals, alcoholates of alkali metals, hydroxides of alkaline earth metals and alcoholates of alkaline earth metals, and distilling off water formed in the course of the reaction from the reaction system.

Preferable group R in formulas (1) and (2) above are those having 3 to 20 carbon atoms. Among them, examples of alkyl include heptyl, nonyl, undecyl, lauryl and myristyl. Alkenyl groups may contain two or more double bonds, and exemplary alkenyls are led by 2,6-dimethyl-1,5-heptadienyl group, 2,6-dimethyl-5-heptenyl group and the like. Examples of aralkyl include 2-phenethyl and 2-styryl, examples of aryl include phenyl, methylphenyl and dimethylphenyl.

Examples of substituents which may be contained in these groups include cyano, hydroxyl, alkoxyl, nitro, alkoxycarbonyl, amide and a halogen atom.

Aldoxime (2) which is a starting compound and is useful in the present invention can be obtained, for example, from the reaction between a corresponding aldehyde and an inorganic salt of hydroxyl amine by a conventional method.

Examples of the catalyst which are useful in the reaction of the present invention include hydroxides of alkali metals, alcoholates of alkali metals, carbonates of alkali metals, bicarbonates of alkali metals, hydroxides of alkaline earth metals, alcoholates of alkaline earth metals, carbonates of alkaline earth metals and bicarbonates of alkaline earth metals. They may be used singly or in combination as desired. In detail, sodium hydroxide, potassium hydroxide, lithium hydroxide, calcium hydroxide, barium hydroxide, magnesium hydroxide, sodium methoxide, sodium ethoxide, potassium propoxide, potassium isoproxide, lithium butoxide, sodium carbonate, potassium carbonate, lithium carbonate, calcium carbonate, magnesium carbonate, sodium hydrogencarbonate, potassium hydrogencarbonate and lithium hydrogencarbonate and the like can be used singly or in combination of two or more.

Among these catalysts, preferable ones are hydroxides of alkali metals, alcoholates of alkali metals, hydroxides of alkaline earth metals and alcoholates of alkaline earth metals in view of the yield, and when production cost is further considered, sodium hydroxide and potassium hydroxide are most preferred.

In the present invention, the preferable amount of catalyst is from 0.1 to 50% by weight, and especially, 0.1 to 5% by weight in total with respect to the starting aldoxime (2). An amount less than 0.1% by weight will result in a low yield of nitrile, and an amount in excess of 50% by weight can no more improve the effect obtainable from the use of catalysts in an amount of 50% by weight or less.

Preferable temperature range for obtaining nitrile (1) from aldoxime (2) according to this invention is from 80 to 200 °C.

No particular limitation is imposed as to the means for distilling off water formed in the course of the reaction from the reaction system, and mention may be given to azeotropic distillation by the use of a solvent which can form an azeotropic mixture with water, and distillation by evaporation under reduced pressure of the reaction system, both promise an effective production.

Examples of solvents which can form an azeotropic mixture with water include benzene, toluene, xylene, chlorobenzene and heptane.

Preferable process for distilling off water by evaporation under reduced pressure is such that aldoxime (2) is supplied to a mixture of a solvent of high-boiling point and one or more of the aforementioned catalysts under reduced pressure at a reaction temperature, followed by distilling off the produced water successively. Here, examples of high-boiling point solvent include those which have a higher boiling point than the reaction product, nitrile (1), such as liquid paraffin and alkylbenzene.

In the present invention, the reaction for obtaining nitrile (1) from aldoxime (2) preferably proceeds, in general, under atmospheric pressure when an azeotropic distillation by the use of the above-mentioned azeotropic solvents is adopted for removing water. On the other hand, when distillation of water by evaporation under reduced pressure by the addition of a high-boiling point solvent is adopted, the reaction preferably proceeds under 200 torr or less, more preferably 60 torr or less, in general.

The thus obtained crude nitrile is purified by evaporation, column chromatography or the like to isolate the target compound, nitrile (1).

## EXAMPLES

This invention will now be described in detail by way of examples, which however, should not be construed as limiting the invention thereto.

Example 1.

Into a 200 ml four-neck flask equipped with a stirrer, a thermometer and a Deanstark dehydrating apparatus, 50 g of 3,7-dimethyl-6-octenoxime, 2 g of potassium hydroxide and 25 ml of toluene were charged. While the water formed is azeotropically distilled off together with toluene under reflux, stirring was continued for 2 hours at 126 °C, followed by cooling down to 30 - 40 °C. Subsequently, the content was neutralized with acetic acid, toluene was distilled off and evaporation was performed to obtain 44.0 g of a fraction of distillate [90 °C/5 mmHg (6,6 mbar)]. Analysis of the obtained fraction revealed the production of 3,7-dimethyl-6-octenonitrile in the purity of 94.5%, yield of 93%, conversion of 99% and selectivity of 94%.

Here, the conversion and selectivity are respectively defined as follows:

$$\text{Conversion (\%)} = \frac{\text{(Weight of charged oxime)} - \text{(Weight of recovered oxime)}}{\text{(Weight of charged oxime)}} \times 100$$

$$\text{Selectivity (\%)} = \frac{\text{(Mols of produced nitrile)}}{\text{(Mols of converted oxime)}} \times 100$$

Example 2.

Into a 200 ml four-neck flask equipped with a stirrer, a thermometer and a Deanstark dehydrating apparatus, 50 g of 3,7-dimethyl-6-octenoxime, 5 g of sodium hydroxide and 25 ml of toluene were charged. While the water formed is azeotropically distilled off together with toluene under reflux, stirring was continued for 2 hours at 125 °C, followed by cooling down to 30 - 40 °C. Subsequently, the content was neutralized with acetic acid, toluene was distilled off and evaporation was performed to obtain 41.2 g of a fraction of distillate [90 °C/5 mmHg (6,6 mbar)]. Analysis of the obtained fraction revealed the production of 3,7-dimethyl-6-octenonitrile in the purity of 95.5%, yield of 88%, conversion of 99% and selectivity of 89%.

Example 3.

Into a 200 ml four-neck flask equipped with a stirrer, a thermometer and a Deanstark dehydrating apparatus, 50 g of 3,7-dimethyl-2,6-octadienoxime, 5 g of potassium hydroxide and 25 ml of toluene were charged. While the water formed is azeotropically distilled off together with toluene under reflux, stirring was continued for 2 hours at 126 °C, followed by cooling down to 30 - 40 °C. Subsequently, the content was neutralized with acetic acid, toluene was distilled off and evaporation was performed to obtain 46.0 g of a fraction of distillate [79 °C/2 mmHg (2,7 mbar)]. Analysis of the obtained fraction revealed the production of 3,7-dimethyl-2,6-octadienonitrile in the purity of 94.5%, yield of 90%, conversion of 99% and selectivity of 91%.

Example 4.

Into a 200 ml four-neck flask equipped with a stirrer, a thermometer and a Deanstark dehydrating apparatus, 50 g of heptanoxime, 2 g of potassium hydroxide and 25 ml of toluene were charged. While the water formed is azeotropically distilled off together with toluene under reflux, stirring was continued for 2

hours at 120°C, followed by cooling down to 30 - 40°C. Subsequently, the content was neutralized with acetic acid, toluene was distilled off and evaporation was performed to obtain 36.8 g of a fraction of distillate [90°C/15 mmHg (20 mbar)]. Analysis of the obtained fraction revealed the production of heptanonitrile in the purity of 98.7%, yield of 83%, conversion of 97% and selectivity of 86.7%.

Comparative Example 1.

Into a 200 ml four-neck flask equipped with a stirrer, a thermometer and a Deanstark dehydrating apparatus, 50 g of 3,7-dimethyl-6-octenoxime, 2.5 g of sulfuric acid and 25 ml of toluene were charged. Under reflux, stirring was performed for 2 hours at 124°C, followed by cooling down to 30 - 40°C. Subsequently, the content was neutralized with sodium hydrogencarbonate, toluene was distilled off and evaporation was conducted to obtain 36.4 g of a fraction of distillate [90°C/5 mmHg (6,6 mbar)]. Analysis of the obtained fraction revealed the production of 3,7-dimethyl-6-octenonitrile in the purity of 7.1%, yield of 6%, conversion of 46.6% and selectivity of 12%. 26.7 g of unreacted oxime was recovered.

Examples 5 - 8.

Procedure of Example 1 was followed using 1 g of a compound shown in Table 1 as a basic catalyst and 50 g of a starting aldoxime as shown in Table 1 to prepare a nitrile. The obtained nitriles, yields, conversions and selectivities are also shown in Table 1.

Table 1

| Ex. No. | Starting Aldoxime | Catalyst | Produced Nitrile | a) | b) | c) |
|---|---|---|---|---|---|---|
| 5 | 3,7-dimethyl-6-octenoxime | sodium methoxide | 3,7-dimethyl-6-octenonitrile | 99 | 77 | 78 |
| 6 | benzoxime | potassium hydroxide | benzonitrile | 99 | 75 | 76 |
| 7 | 3-phenylpropanoxime | potassium hydroxide | 3-phenylpropanonitrile | 99 | 85 | 86 |
| 8 | 3-phenyl-2-propenoxime | potassium hydroxide | 3-phenyl-2-propenonitrile | 99 | 80 | 81 |

a): Conversion (%)
b): Yield (%)
c): Selectivity (%)

Example 9.

Into a 300 ml flask equipped with a thermometer, stirrer, charging tube and a distillating tube, 30 g of liquid paraffin and 6 g of KOH were placed, stirred, heated at 110 - 120°C, and the pressure was controlled to be 50 torr. Subsequently, 3,7-dimethyl-2,6-octadienoxime was supplied at a rate of 100 g/hour over 3 hours, and aged for 1 hour under the same conditions to obtain 258 g of an oil distillate together with a small amount of water. Analysis of this fraction revealed that 3,7-dimethyl-2,6-octadienonitrile was produced in the purity of 93.5% and yield of 90.0%.

**INDUSTRIAL APPLICABILITY**

According to the process of the present invention, a nitrile which is useful as a perfume or as a starting material in the synthesis of perfumes, medicines and so on can be obtained in high yields and at a remarkably advantageous production cost. Wide range of application of the present process, therefore, is expected in various fields of the perfume manufacture, medicine manufacture and the like.

**Claims**

**1.** A process for producing a nitrile represented by the general formula (1):

$RC \equiv N$ (1)

wherein R is alkyl, alkenyl, aralkyl or aryl, each may have a substituent, characterized by heating an

aldoxime represented by the general formula (2):

RCH = NOH (2)

wherein R is as defined above, in the presence of one or more catalysts selected from hydroxides of alkali metals, alcoholates of alkali metals, hydroxides of alkaline earth metals and alcoholates of alkaline earth metals, and distilling off water formed in the course of the reaction from the reaction system.

**2.** The process for producing a nitrile as defined in Claim 1, wherein the catalyst is sodium hydroxide and/or potassium hydroxide.

**3.** The process for producing a nitrile as defined in Claim 1 or 2, wherein the aldoxime is 3,7-dimethyl-2,6-octadienoxime.

**4.** The process for producing a nitrile as defined in Claim 1 or 2, wherein the aldoxime is 3,7-dimethyl-6-octenoxime.

**5.** The process for producing a nitrile as defined in Claim 1, wherein the distillation of water formed in the course of the reaction is an azeotropic distillation by the use of a solvent which is capable of forming an azeotropic mixture with water.

**6.** The process for producing a nitrile as defined in Claim 1, wherein the distillation of water formed in the course of the reaction is evaporation under reduced pressure of the reaction system.

**7.** The process for producing a nitrile as defined in Claim 6, wherein the evaporation is carried out by supplying aldoxime into a mixture solution of a high-boiling point solvent and a catalyst under reduced pressure at a reaction temperature, then water formed is distilled off.

**Patentansprüche**

**1.** Verfahren zur Herstellung eines Nitrils, das durch die allgemeine Formel (1) dargestellt ist:

RC ≡ N (1)

in der R Alkyl, Alkenyl, Aralkyl oder Aryl ist, wobei jeder Rest einen Substituenten tragen kann, dadurch gekennzeichnet, daß ein Aldoxim, das durch die allgemeine Formel (2) dargestellt ist:

RCH = NOH (2)

in der R wie oben definiert ist, in der Gegenwart von einem oder mehreren Katalysatoren, ausgewählt aus Hydroxiden von Alkalimetallen, Alkoholaten von Alkalimetallen, Hydroxiden von Erdalkalimetallen und Alkoholaten von Erdalkalimetallen, erhitzt wird und daß Wasser, das im Verlauf der Reaktion gebildet wird, aus dem Reaktionsssystem abdestilliert wird.

**2.** Verfahren zur Herstellung eines Nitrils, wie in Anspruch 1 definiert, dadurch gekennzeichnet, daß der Katalysator Natriumhydroxid und/oder Kaliumhydroxid ist.

**3.** Verfahren zur Herstellung eines Nitrils, wie in Anspruch 1 oder 2 definiert, dadurch gekennzeichnet, daß das Aldoxim 3,7-Dimethyl-2,6-octadienoxim ist.

**4.** Verfahren zur Herstellung eines Nitrils, wie in Anspruch 1 oder 2 definiert, dadurch gekennzeichnet, daß das Aldoxim 3,7-Dimethyl-6-octenoxim ist.

**5.** Verfahren zur Herstellung eines Nitrils, wie in Anspruch 1 definiert, dadurch gekennzeichnet, daß die Destillation von Wasser, das im Verlauf der Reaktion gebildet wird, eine azeotrope Destillation unter Verwendung eines Lösungsmittels, das mit Wasser eine azeotrope Mischung zu bilden vermag, ist.

6. Verfahren zur Herstellung eines Nitrils, wie in Anspruch 1 definiert, dadurch gekennzeichnet, daß die Destillation von Wasser, das im Verlauf der Reaktion gebildet wird, Verdampfung unter vermindertem Druck des Reaktionssystems ist.

7. Verfahren zur Herstellung eines Nitrils, wie in Anspruch 6 definiert, dadurch gekennzeichnet, daß die Verdampfung ausgeführt wird, indem Aldoxim in eine Lösungsmischung eines Lösungsmittels mit hohem Siedepunkt und eines Katalysators unter vermindertem Druck bei einer Reaktionstemperatur eingeführt wird, und dann gebildetes Wasser abdestilliert wird.

## Revendications

1. Procédé de production d'un nitrile représenté par la formule générale (1) :

$$RC \equiv N \qquad (1)$$

dans laquelle R représente un groupe alkyle, alcényle, aralkyle ou aryle, chacun d'eux pouvant porter un substituant, caractérisé en ce que l'on chauffe une aldoxime représentée par la formule générale (2) :

$$RCH = NOH \qquad (2)$$

dans laquelle R est tel que défini ci-dessus, en présence d'un ou plusieurs catalyseurs choisis parmi des hydroxydes de métaux alcalins, des alcoolates de métaux alcalins, des hydroxydes de métaux alcalino-terreux et des alcoolates de métaux alcalino-terreux, et en ce qu'on élimine par distillation du système de réaction l'eau formée au cours de la réaction.

2. Procédé de production d'un nitrile selon la revendication 1, dans lequel le catalyseur est de l'hydroxyde de sodium et/ou de l'hydroxyde de potassium.

3. Procédé de production d'une nitrile selon la revendication 1 ou 2, dans lequel l'aldoxime est la 3,7-diméthyl-2,6-octadiènoxime.

4. Procédé de production d'un nitrile selon la revendication 1 ou 2, dans lequel l'aldoxime est la 3,7-diméthyl-6-octènoxime.

5. Procédé de production d'un nitrile selon la revendication 1, dans lequel la distillation de l'eau formée au cours de la réaction est une distillation azéotropique obtenue en utilisant un solvant qui est capable de former un mélange azéotropique avec l'eau.

6. Procédé de production d'un nitrile selon la revendication 1, dans lequel la distillation de l'eau formée au cours de la réaction est une évaporation sous pression réduite du système de réaction.

7. Procédé de production d'un nitrile selon la revendication 6, dans lequel on effectue l'évaporation en introduisant une aldoxime dans une solution d'un mélange d'un solvant de point d'ébullition élevé et d'un catalyseur sous pression réduite à une température de réaction, puis on élimine l'eau formée par distillation.